# EUROPÄISCHE PATENTSCHRIFT

(11) **EP 2 089 414 B1**
(45) Veröffentlichungstag und Bekanntmachung des Hinweises auf die Patenterteilung: **06.03.2013**
(21) Anmeldenummer: 07819078.2
(22) Anmeldetag: 18.10.2007
(51) Int. Cl.: C07K 1/14

(54) **VERWENDUNG VON IONISCHEN FLÜSSIGKEITEN ZUR MEMBRANPROTEINEXTRAKTION**
USE OF IONIC LIQUIDS FOR MEMBRANE PROTEIN EXTRACTION
UTILISATION DE LIQUIDES IONIQUES POUR L'EXTRACTION DE PROTÉINE MEMBRANAIRE

(30) Priorität: 17.11.2006 DE 102006054329
(43) Veröffentlichungstag der Anmeldung: 19.08.2009
(73) Patentinhaber: Merck Patent GmbH, 64293 Darmstadt (DE)
(72) Erfinder: VON HAGEN, Joerg, 64319 Pfungstadt (DE); MICHELSEN, Uwe, 69469 Weinheim (DE); WEHSLING, Maria, 64285 Darmstadt (DE)
(86) Internationale Anmeldenummer: PCT/EP2007/009010
(87) Internationale Veröffentlichungsnummer: WO 2008/058604

(56) Entgegenhaltungen:
- EP-A- 1 734 047
- CN-A- 1 807 446
- US-A1- 2004 031 685
- RUTH MARIAH C ET AL: "Analysis of membrane proteins from human chronic myelogenous leukemia cells: Comparison of extraction methods for multidimensional LC-MS/MS" JOURNAL OF PROTEOME RESEARCH, Bd. 5, Nr. 3, März 2006 (2006-03), Seiten 709-719, XP002469140 ISSN: 1535-3893

## Beschreibung

Die vorliegende Erfindung betrifft die Verwendung von Ionischen Flüssigkeiten oder von Mischungen enthaltend mindestens eine Ionische Flüssigkeit und mindestens ein weiteres Lösungsmittel zur Extraktion von Membranproteinen aus biologischen Proben, Verfahren zur Extraktion von Membranproteinen, ein Kit zur Extraktion dieser Proteine sowie dessen Verwendung.

Der Nachweis beziehungsweise die Analyse von Proteinen, ganz besonders von Membranproteinen, gewinnt in der Medizin zunehmende Bedeutung. Die Mehrheit der in der pharmazeutischen Forschung untersuchten Systeme beinhalten Membranproteine. Membranproteinen kommt bei einer Reihe biologischer Funktionen eine besondere Bedeutung zu. So spielen viele Membranproteine bei der Entstehung von Krankheiten eine große Rolle, so dass das Verständnis ihrer Funktion bei Entwicklung von Medikamenten zunehmende Wichtigkeit erlangt. Daher ist die Information über die strukturelle Eigenschaft und über die Funktion dieser Proteine Grundlage für das Verständnis der Mechanismen.

Membranproteine, insbesondere Transmembranproteine, besitzen hydrophobe Regionen und sind damit in Membranen verankert und somit schwer löslich in Wasser. Um eine in-vitro-Analyse der Proteine zu ermöglichen, werden Membranproteine üblicherweise durch Zusatz von Detergentien solubilisiert. Die Isolierung von Membranproteinen mit Detergentien hat aber den gravierenden Nachteil, dass die native Struktur der Proteine durch den Einfluss des Detergens denaturiert wird. Gängige Detergentien sind entweder ionischer Natur, wie beispielsweise Natriumdodecylsulfat (SDS), oder nicht-ionischer Natur, wie beispielsweise Triton-X 100. Der Einsatz von SDS führt zu einer vollständigen Denaturierung aller Proteine und damit auch der Membranproteine, das heißt strukturelle und funktionelle Untersuchungen an den Membranproteinen sind nicht oder nur sehr stark eingeschränkt möglich. Triton-X 100 ist nicht in der Lage Membranproteine, insbesondere multiple transmembranöse Proteine, effektiv zu extrahieren, so dass die gewünschten Untersuchungen gar nicht erst erfolgen können.

In US 2004/031685 wird ein Verfahren zur Solubilisierung von Membranproteinen mittels ionischer Flüssigkeiten offenbart. Die ionische Flüssigkeit ist dabei in organischen Lösungsmitteln gelöst.

CN 1807446A offenbart ein Verfahren zur Extraktion von Proteinen unter Verwendung von Extraktionsmitteln, die einen hohen Anteil an ionischen Flüssigkeiten enthalten. Die Extraktion von Membranproteinen wird nicht erwähnt.

Es bestand daher die Aufgabe Verfahren bereit zu stellen, mit deren Hilfe Membranproteine aus biologischen Proben extrahiert werden können, um sie weiter analysieren zu können.

Gegenstand der vorliegenden Erfindung ist demgemäss die Verwendung von Mischungen enthaltend mindestens Wasser und zwischen 0.02 und 5 Gew.% einer Ionischen Flüssigkeit zur Extraktion von Membranproteinen aus biologischen Proben, wobei die ionische Flüssigkeit ausgewählt ist aus N-(3-Hydroxypropyl)-N-methylmorpholiniumbistrifluormethysulfonylimid, 4-(2-Methoxy-ethyl)-4-methyl-morpholiniumbromid, 4-Methyl-4-propyl-morpholiniumbromid, 4-(2-Ethoxy-ethyl)-4-methyl-morpholiniumbromid, 4-(3-Hydroxy-propyl)-4-methyl-morpholiniumbromid, 4-(2-Hydroxy-ethyl)-4-methyl-morpholiniumbromid, 4-(3-Methoxy-propyl)-4-methylmorpholiniumbromid, 4-Butyl-4-propyl-morpholiniumbromid, Trihexyl(tetradecyl)-phosphonium-tetrafluoroborat, 1-Decyl-3-methyl-imidazoliumbromid, 1-Dodecyl-3-methyl-imidazoliumchlorid, 3-Methyl-1-octadecyl-imidazolium-hexafluorophosphat oder Mischungen hieraus.

In einer weiteren bevorzugten Ausführungsform handelt es sich bei den Membranproteinen um Proteine, die zwei oder mehr Transmembranpassagen aufweisen.

In einer weiteren bevorzugten Ausführungsform handelt es sich bei den biologischen Proben um Gewebe, Zellen, Zellkulturen und/oder Körperflüssigkeiten, Bakterien, Pilzen, Viren oder Pflanzen. Gegenstand der vorliegenden Erfindung ist auch ein Verfahren zur Extraktion von Membranproteinen aus bevorzugt nativen, biologischen Proben, dadurch gekennzeichnet, dass eine bevorzugt native, biologische Probe mit einer Mischung, enthaltend mindestens Wasser und zwischen 0.02 und 5 Gew.% einer Ionischen Flüssigkeit, gegebenenfalls unter mechanischer Einwirkung, versetzt wird, wobei die ionische Flüssigkeit ausgewählt ist aus N-(3-Hydroxypropyl)-N-methylmorpholiniumbistrifluormethysulfonylimid, 4-(2-Methoxy-ethyl)-4-methyl-morpholiniumbromid, 4-Methyl-4-propyl-morpholiniumbromid, 4-(2-Ethoxy-ethyl)-4-methyl-morpholiniumbromid, 4-(3-Hydroxy-propyl)-4-methyl-morpholiniumbromid, 4-(2-Hydroxy-ethyl)-4-methylmorpholiniumbromid, 4-(3-Methoxy-propyl)-4-methyl-morpholiniumbromid, 4-Butyl-4-propyl-morpholiniumbromid, Trihexyl(tetradecyl)-phosphonium-tetrafluoroborat, 1-Decyl-3-methyl-imidazoliumbromid, 1-Dodecyl-3-methyl-imidazoliumchlorid, 3-Methyl-1-octadecyl-imidazolium-hexafluorophosphat oder Mischungen hieraus.

In einer bevorzugten Ausführungsform wird die biologische Probe vorher lysiert.

In einer besonders bevorzugten Ausführungsform erfolgt das Lysieren der biologischen Proben durch Zusatz von Detergenzien, oberflächenaktiven Substanzen oder Porenbildnern.

In einer bevorzugten Ausführungsform erfolgt die mechanische Einwirkung durch Schütteln oder Rühren.

In einer weiteren bevorzugten Ausführungsform erfolgt die Extraktion bei Temperaturen von 4 bis 37°C.

Kern der vorliegenden Erfindung ist, dass das erfindungsgemäße Verfahren geeignet ist, Membranproteine, insbesondere mehrfach membrangängige Proteine, schonend und möglichst strukturerhaltend zu extrahieren. Dem Fachmann ist bekannt, dass insbesondere bei mehrfach membrangängigen Proteinen eine funktionserhaltende Extraktion aus der Membran kaum möglich ist, da sich die 3-D Struktur des Proteins nach der Extraktion aus der Membran zwangsläufig ändert, wenn die Transmembrandomänen aus der hydrophoben Umgebung der Membran entfernt werden. Daher bedeutet im Hinblick auf die erfindungsgemäße Extraktion von mehrfach membrangängigen Proteinen, der Begriff "nativ", dass die extrahierten Membranproteine zwar in der Regel nicht funktionserhaltend, wohl aber schonend und möglichst strukturerhaltend extrahiert werden. Beispielsweise ermöglicht die erfindungsgemäße Extraktion die massenspektrometrische und immunologische Messung insbesondere der Transmembrandomänen der Proteine. Das ist mit herkömmlichen Methoden wie z.B. der Extraktion mit Triton-X100, Nonidet P40 oder anderen Detergenzien die standardmäßig Anwendung finden in Bezug auf die Proteinausbeute und Funktionserhalt der zu untersuchenden Proteine nicht vergleichbar möglich.

Bei Membranproteinen, die nur mit einem für ihre Funktion bzw. Aktivität irrelevanten Teil in der Membran verankert sind, wie z.B. GPI-Anker-Proteine, bedeutet die erfindungsgemässe "native" Extraktion, dass das Protein weitgehend struktur- und aktivitätserhaltend extrahiert werden kann. Beispielsweise können in diesem Fall zum Nachweis des Proteins entsprechende Aktivitätsmessungen durchgeführt werden.

Native Proben sind Proben, in denen die zu extrahierenden Membranproteine noch weitgehend in ihrer nativen, d.h. in der für ihre natürliche Funktion notwendigen Konformation vorliegen, bzw Proben, in denen die Membranproteine noch Aktivität zeigen.

Die Extraktion von Membranproteinen gemäß der vorliegenden Erfindung kann aus allen dem Fachmann bekannten biologischen Proben erfolgen. Vorzugsweise handelt es sich bei den biologischen Proben um Gewebe, wie z.B. Biopsien und histologische Präparate, Zellen, Zellkulturen und/oder zellhaltige Körperflüssigkeiten, wie z.B. Blut, Urin, Liquor oder Speichel,sowie Bakterien, Pflanzen und Pilze. Auch Membranproteine aus membranhaltigen Zellkompartimenten können erfindungsgemäß extrahiert werden. Die Extraktion von Proteinen aus Geweben und Zellkulturen erlaubt insbesondere den Nachweis spezifischer Proteine, z.B. zum Nachweis von Proteinen, die auf das Vorhandensein von Krankheiten schließen lassen. Daher ist das erfindungsgemäße Verfahren insbesondere auch für pathologisch interessante Gewebeproben vorteilhaft.

Das erfindungsgemäße Verfahren ist insbesondere geeignet für Transmembranproteine und in ganz besonderer Weise für mehrfach membrangängige Proteine, d.h. Proteine, die zwei oder mehr Transmembranpassagen aufweisen, insbesondere multihelikale Transmembranproteine, wie z.B. heptahelikale Transmembranproteine. Die Klasse der heptahelikalen Transmembranproteine umfasst derzeit ca 250 bekannte Proteine. Die Transmembranproteine können hierbei in folgende Subklassen eingeteilt werden:
- Class A Rhodopsine, Hormonproteine, (Rhod)opsin, Olfactory, Prostanoide, Nucleotid-Analoga, Cannabinoid, Platelet activating factor, Gonadotropin-freisetzende Hormone, Thyrotropin-freisetzende Hormone und Secretagogue, Melatonin, virale Proteine, Lysosphingolipid & LPA (EDG), Leukotriene B4 Rezeptoren, Class A Orphan und andere,
- Class B Secretine, beispielsweise Calcitonin, Corticotropin releasing factor, Gastric inhibitory peptide, Glucagon, Growth hormone-releasing Hormone, Parathyroid Hormone, PACAP, Secretin, Vasoactive intestinale Polypeptide, Diuretic Hormone, EMR1, Latrophilin, Brainspecific angiogenesis inhibitor (BAI), Methuselah-like proteins (MTH), Cadherin EGF LAG (CELSR), Very large G-protein coupled Rezeptoren
- Class C Metabotropic glutamate/pheromone, beispielsweise Metabotropic glutamate, Calcium-sensing like, Putative pheromone receptors, GABA-B, Orphan GPRC5, Orphan GPCR6, Bride of sevenless proteins (BOSS), Taste receptors (T1 R),
- Class D Fungal pheromone, beispielsweise Fungal pheromone A-Factor like (STE2,STE3), Fungal pheromone B like (BAR,BBR,RCB,PRA), Fungal pheromone M- and P-Factor, Class E cAMP receptors, Frizzled/Smoothened family, frizzled, Smoothened und in die folgenden Non-GPCR-Familien: Class Z Archaeal/bacterial/fungal opsins.

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf.

Das Gebiet der ionischen Flüssigkeiten wird zurzeit intensiv erforscht, da die Anwendungsmöglichkeiten vielfältig sind. Übersichtsartikel zu ionischen Flüssigkeiten sind beispielsweise R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse", Angew. Chem., 112 (2000), 3926-3945; T. Welton "Room temperature ionic liquids. Solvents for synthesis and catalysis", Chem. Rev., 92 (1999), 2071-2083 oder R. Hagiwara, Ya. Ito "Room temperature ionic liquids of alkylimidazolium cations and fluoroanions", J. Fluorine Chem., 105 (2000), 221-227).

Die erfindungsgemäß einzusetzenden Ionischen Flüssigkeiten sind mit Wasser mischbar. Sie werden gemäß der vorliegenden Erfindung in einer Mischung enthaltend mindestens eine Ionische Flüssigkeit und mindestens ein weiteres Lösungsmittel eingesetzt werden. Bei dem weiteren Lösungsmittel handelt es sich um Wasser oder bevorzugt um wässrige Puffersysteme. Dem Lösungsmittel können auch kleinere Mengen eines oder mehrerer mit Wasser löslicher organischer Lösungsmittel zugesetzt sein.

In der einfachsten Ausführungsform der vorliegenden Erfindung wird die biologische Probe mit einer Mischung enthaltend Wasser und mindestens eine Ionische Flüssigkeit versetzt. Vorzugsweise erfolgt das genannte Verfahren unter mechanischer Einwirkung, beispielsweise durch Schütteln oder Rühren. Auf diese Weise wird die Extraktion der Membranproteine beschleunigt und die Ausbeute an extrahierten Proteinen verbessert.

In einer weiteren Ausführungsform des erfindungsgemäßen Verfahrens kann die biologische Probe zunächst lysiert werden, d.h. die zelluläre Grundstruktur vor Anwendung der erfindungsgemäßen Verfahren wurde zerstört. Diese Vorbehandlung kann auf alle dem Fachmann bekannten Arten erfolgen, z.B. durch manuelle Homogenisierung, mechanisches Schütteln.

Insbesondere kann das Lysieren der biologischen Proben auch durch Zusatz von dem Fachmann bekannten Detergenzien, oberflächenaktive Substanzen und Porenbildner erfolgen. Durch das vorherige Lysieren der Probe wird das Ergebnis der Extraktion hinsichtlich der Ausbeute an erhaltenen Membranproteinen nochmals verbessert. So verbleiben beim Lysieren die Membranproteine in der Membran, die Membranen bzw. Membranfragmente können aber in einfacher Weise von den anderen Zellbestandteilen abgetrennt werden. Bevorzugt erfolgt die Lyse mit Digitonin.

Die Lyse kann auch gleichzeitig mit der Extraktion durchgeführt werden, dann erhält man jedoch komplexere Proteingemische.

Generell eignen sich in den erfindungsgemäßen Verfahren alle Ionischen Flüssigkeiten gemäß Anspruch 1.

N-(3-Hydroxypropyl)-N-methylmorpholiniumbistrifluormethysulfonylimid, 4-(2-Methoxy-ethyl)-4-methyl-morpholiniumbromid, 4-Methyl-4-propyl-morpholiniumbromid, 4-(2-Ethoxy-ethyl)-4-methyl-morpholiniumbromid, 4-(3-Hydroxy-propyl)-4-methyl-morpholiniumbromid, 4-(2-Hydroxy-ethyl)-4-methyl-morpholiniumbromid, 4-(3-Methoxy-propyl)-4-methylmorpholiniumbromid, 4-Butyl-4-propyl-morpholiniumbromid, Trihexyl(tetradecyl)-phosphonium-tetrafluoroborat, 1-Decyl-3-methyl-imidazoliumbromid, 1-Dodecyl-3-methyl-imidazoliumchlorid, 3-Methyl-1-octadecyl-imidazolium-hexafluorophosphat oder Mischungen hieraus liefern besonders gute Ergebnisse bei den erfindungsgemäßen Verfahren.

Die Konzentration der Ionischen Flüssigkeit in der Mischung enthaltend mindestens eine Ionische Flüssigkeit und mindestens ein weiteres Lösungsmittel beträgt 0.02 bis 5 Gew.-%, bevorzugt 0,1 bis 1 Gew%, bezogen auf die Mischung. Geeignete weitere Lösungsmittel sind Wasser oder wässrige Puffersysteme. Geeignete Puffer sind alle Puffersysteme, die physiologische Bedingungen erzeugen, d.h. Proteine nicht denaturieren. Beispiele sind PIPES, HEPES, Phosphatpuffer und Tris basierte Puffer.

In den genannten Verfahren können die Mischungen enthaltend mindestens eine Ionische Flüssigkeit und mindestens ein weiteres Lösungsmittel zusätzliche Additive und Hilfsstoffe enthalten. Entsprechende Additive und Hilfsstoffe sind dem Fachmann bekannt und umfassen beispielsweise Detergenzien, oberflächenaktive Substanzen, Porenbildner und biologische bzw. physiologische Puffersysteme, Mineralsalze und Inhibitoren (beispielsweise Proteaseinhibitoren).

Die erfindungsgemäßen Verfahren können bei Temperaturen oberhalb von von 0°C durchgeführt werden, typischerweise zwischen 0 und 95°C . Wenn insbesondere hohe Proteinausbeuten erzielt werden sollen und der Erhalt von Aktivität oder Struktur sekundär sind, kann die Extraktion bei hohen Temperature (über 37°C) durchgeführt werden. Derartige Extraktionen können besonders gut für Western Blot Analysen genutzt werdenvorzugsweise erfolgt die erfindungsgemäße Extraktion bei 0 bis 37°C, besonders bevorzugt zwischen 0 und 8 °C, insbesondere zwischen 0 und 4°C. Bei den bevorzugten Temperaturen wird eine verbesserte Extraktion in relativ kurzer Zeit und die Beibehaltung der Proteinaktivität des Proteins beobachtet. Zur schonenden Extraktion, insbesondere von empfindlicheren Membranproteinen empfiehlt sich die Durchführung des erfindungsgemäßen Verfahrens bei tieferen Temperaturen innerhalb des angegebenen Temperaturintervalls, unter Berücksichtigung einer dafür erforderlichen längeren Extraktionszeit.

Typische Extraktionszeiten liegen zwischen 30 Minuten und 16 Stunden. Wenn aktive Proteine extrahiert werden sollen, sollte der pH-Wert der Extraktionslösung (Lösungsmittel und ionische Flüssigkeit) bevorzut bei ca. pH 7.4 liegen, ansonsten können auch Extraktionslösungen mit pH-Werten zwischen 2 und 10 eingesetzt werden.

Die erfindungsgemäß extrahierten Proteine können beispielsweise für massenspektrometrische Untersuchungen direkt oder nach gelelektrophoretischer Auftrennung, für Western Blot Analysen oder Aktivitätsassays eingesetzt werden. Für einige Anwendungen, wie z.B. massenspektrometrische Bestimmung muss die ionische Flüssigkeit vorher aus dem erhaltenen Proteinextrakt entfernt werden. Das kann beispielsweise durch in gel Verdau nach dem Fachmann bekannten Verfahren erfolgen.

Die erfindungsgemäßen Verfahren eignen sich zur Extraktion von Membranproteinen aus biologischen Proben unter Erhalt der zellulären Grundstruktur der Proben, d.h. die Struktur der Membranproteine bleibt soweit wie möglich erhalten. Auf diese Weise lassen sich auch ganze Membrankomplexe isolieren, die mit den herkömmlichen Methoden nicht oder nur schwer isolierbar sind. In der Regel lassen sich die erhaltenen Membranproteine mit geeigneten Antikörpern nachweisen

Das erfindungsgemäße Verfahren eröffnet erstmals die Möglichkeit, hydrophobe Membranproteine, insbesondere mehrfach membrangängige Proteine, in einem wässrigen System unter Anwendung von wasserlöslichen ionischen Flüssigkeiten als milde Extraktionsmittel zu extrahieren. Auf diese Weise wird eine schonende Extraktion kombiniert mit einer sehr einfachen Verfahrensführung. Da bereits für die Extraktion ein wässriges Medium verwendet wird, kann der Extrakt direkt in verschiedensten Analyseverfahren untersucht werden, ohne vorher das Lösungsmittel zu tauschen.

Die mit Hilfe der erfindungsgemäßen Verfahren gewonnenen Extrakte enthalten Membranproteine und sind ebenfalls Gegenstand der vorliegenden Erfindung. Diese eignen sich für die Verwendung bei allen dem Fachmann bekannten Arten von Proteinanalytik, z.B. Elektrophorese (z.B. Gelelektrophorese, insbesondere auch 2-dimensionale Gelelektrophorese), immunchemische Nachweismethoden (z.B. Western Blot Analyse, ELISA, RIA), Protein-Arrays (z.B planare und beadbasierende Systeme), Massenspektrometrie (z.B. Maldi, Esi und Seldi) sowie alle chromatographischen Trennverfahren, insbesondere biochromatographischen Trennverfahren (IEX, SEC, HIC, Affinitätschromatographie und Hydrophobe Interaktionschromatographie).

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiele:

Humane MDA MB 468 Brustkrebszellen werden in RPMI 1640 Medium (2,000 mg/L D-Glucose, 110 mg/L Natriumpyruvat, nicht-essentielle Aminosäuren, ohne L-Glutamin, Fa. Gibco) bis zu einer Konfluenz von 50% in handelsüblichen T75 Zellkulturflaschen unter 5% CO₂ bei 37°C kultiviert. Die adhärenten Zellen werden mittels Zellschaber in Tris-gepufferter Kochsalzlösung (TBS) und Proteaseinhibitoren (Calbiochem Protease Inhibitor Cocktail III) geerntet. Die Membranproteine werden nach der Freisetzung der zytoplasmatischen Proteinfraktion mittels Porenbildner (*Extraktionspuffer I*) im 2. Extraktionsschritt isoliert. Die Fraktion 1 repräsentiert die lösliche Proteinfraktion vornehmlich der zytoplasmatischen Proteine. Die Fraktion nach Extraktion mittels Ionischer Flüssigkeit in wässriger Pufferlösung (*Extraktionspuffer II*) ist die Membranproteinfraktion. Es werden 2%-ige Lösungen der Ionischen Flüssigkeit für 30 Minuten bei 37°C unter leichtem Schütteln inkubiert. Nach der Extraktion werden die Proben 15 Minuten bei 16000 x g bei Raumtemperatur zentrifugiert. Der Überstand (extrahierte Membranproteinfraktion) wird anschließend mit handelsüblichem Probenauftragspuffer für die Natriumdodecylsufat Polyacrylamidgelelektrophorese (SDS-PAGE) eingesetzt. Die Proben werden auf 10% BIS-Tris Polyacrylamid (PAA) Gelen (Novex, Invitrogen) aufgetrennt. Anschließend werden die Proben auf PVDF Membranen nach dem Prinzip des "semi-dry Western Blot" Verfahrens transferiert. Anschließend werden die Membranen mit folgenden Erstantikörpern inkubiert:
   a) anti-EGFR rabbit Antikörper (Sc03, Santa Cruz Verdünnung 1:100) als Referenz eines Dimeren Reczeptormoleküls,
   b) Anti-Frizzled 4 Antikörper (R&D Systems MAB 194, diluted 1:500) als Referenz eines siebenfach transmembran passierenden Proteins (7-TM). c) Als zweites Referenzprotein wird Cadherin EGF-lag seven-transmembranepassing receptor-3 (CELSR3) Antikörper verwendet (Acris).
   d) Als Zweitantikörper zur Detektion werden ein anti-Rabbit -POD Antikörper von General Electrics (GE) 1:5000 verdünnt eingesetzt) oder ein anti-Rat-POD Antikörper von Pierce in einer Verdünnung von 1:5000 verwendet.

Die Chemilumineszenzdetektion erfolgt mittels eines handelsüblichen ECL-Western blotting detection kit von GE (#RPN 2106).

Der Western Blot zeigt den Erhalt der nativen Struktur der mit der Ionischen Flüssigkeit extrahierten Membranproteine.
*TBS:* 50 mM Tris
150 mM NaCl
pH 7,4

### Extraktionspuffer I:

300 m Saccharose
15 mM NaCl
10 mM Pipes (Piperazin-1,4-bis(2-ethansulfonsäure))
0,5 mM EDTA
0,01875 % Digitonin
pH 7,4

### Extraktionspuffer II:

300 mM Saccharose
15 mM NaCl
10 mM Pipes (Piperazin-1,4-bis(2-ethansulfonsäure))
0,5 mM EDTA
2Gew % N-(3-hydroxypropyl)-N-methylmorpholinium-bis-(trifluoromethylsulfonyl)imid oder 1-Dodecyl-3-methyl-imidazoliumchlorid
pH 7,4

## Patentansprüche

1. Verwendung von Mischungen enthaltend mindestens Wasser und zwischen 0.02 und 5 Gew.% einer Ionische Flüssigkeit zur Extraktion von Membranproteinen aus biologischen Proben, **dadurch gekennzeichnet, dass** die ionische Flüssigkeit ausgewählt ist aus N-(3-Hydroxypropyl)-N-methylmorpholiniumbistrifluormethysulfonylimid, 4-(2-Methoxy-ethyl)-4-methyl-morpholiniumbromid, 4-Methyl-4-propyl-morpholiniumbromid, 4-(2-Ethoxy-ethyl)-4-methyl-morpholiniumbromid, 4-(3-Hydroxy-propyl)-4-methyl-morpholiniumbromid, 4-(2-Hydroxy-ethyl)-4-methylmorpholiniumbromid, 4-(3-Methoxy-propyl)-4-methyl-morpholiniumbromid, 4-Butyl-4-propyl-morpholiniumbromid, Trihexyl(tetradecyl)-phosphonium-tetrafluoroborat, 1-Decyl-3-methyl-imidazoliumbromid, 1-Dodecyl-3-methyl-imidazoliumchlorid, 3-Methyl-1-octadecyl-imidazolium-hexafluorophosphat oder Mischungen hieraus.

2. Verwendung nach Anspruch 1, **dadurch gekennzeichnet, dass** es sich bei den Membranproteinen um Proteine handelt, die zwei oder mehr Transmembranpassagen aufweisen.

3. Verwendung nach Anspruch 1 oder 2, **dadurch gekennzeichnet, dass** es sich bei den biologischen Proben um Gewebe, Zellen, Zellkulturen und/oder Körperflüssigkeiten, Bakterien, Pilzen, Viren oder Pflanzen handelt.

4. Verfahren zur Extraktion von Membranproteinen aus biologischen Proben, **dadurch gekennzeichnet, dass** eine biologische Probe mit einer Mischung enthaltend mindestens Wasser und zwischen 0.02 und 5 Gew.-% einer Ionische Flüssigkeit, gegebenenfalls unter mechanischer Einwirkung, versetzt wird, **dadurch gekennzeichnet, dass** die ionische Flüssigkeit ausgewählt ist aus N-(3-Hydroxypropyl)-N-methylmorpholiniumbistrifluormethysulfonylimid, 4-(2-Methoxy-ethyl)-4-methyl-morpholiniumbromid, 4-Methyl-4-propyl-morpholiniumbromid, 4-(2-Ethoxy-ethyl)-4-methyl-morpholiniumbromid, 4-(3-Hydroxy-propyl)-4-methyl-morpholiniumbromid, 4-(2-Hydroxy-ethyl)-4-methylmorpholiniumbromid, 4-(3-Methoxy-propyl)-4-methyl-morpholiniumbromid, 4-Butyl-4-propyl-morpholiniumbromid, Trihexyl(tetradecyl)-phosphonium-tetrafluoroborat, 1-Decyl-3-methyl-imidazoliumbromid, 1-Dodecyl-3-methyl-imidazoliumchlorid, 3-Methyl-1-octadecyl-imidazolium-hexafluorophosphat oder Mischungen hieraus.

5. Verfahren nach Anspruch 4, **dadurch gekennzeichnet, dass** die biologische Probe vorher lysiert wird.

6. Verfahren nach Anspruch 4 oder 5, **dadurch gekennzeichnet, dass** das Lysieren der biologischen Proben du rch Zusatz von Detergenzien, oberflächenaktiven Substanzen oder Porenbildnern erfolgt.

7. Verfahren nach einem oder mehreren der Ansprüche 4 bis 6, **dadurch gekennzeichnet, dass** die mechanische Einwirkung durch Schütteln oder Rühren erfolgt.

8. Verfahren nach einem oder mehreren der Ansprüche 4 bis 7, **dadurch gekennzeichnet, dass** die Extraktion bei Temperaturen von 4 bis 37°C erfolgt.

## Claims

1. Use of mixtures comprising at least water and between 0.02 and 5% by weight of an ionic liquid for the extraction of membrane proteins from biological samples, **characterised in that** the ionic liquid is selected from N-(3-hydroxypropyl)-N-methylmorpholinium bistrifluoromethylsulfonylimide, 4-(2-methoxyethyl)-4-methylmorpholinium bromide, 4-methyl-4-propylmorpholinium bromide, 4-(2-ethoxyethyl)-4-methylmorpholinium bromide, 4-(3-hydroxypropyl)-4-methylmorpholinium bromide, 4-(2-hydroxyethyl)-4-methylmorpholinium bromide, 4-(3-methoxypropyl)-4-methylmorpholinium bromide, 4-butyl-4-propylmorpholinium bromide, trihexyl(tetradecyl)phosphonium tetrafluoroborate, 1-decyl-3-methylimidazolium bromide, 1-dodecyl-3-methylimidazolium chloride, 3-methyl-1-octadecylimidazolium hexafluorophosphate or mixtures thereof.

2. Use according to Claim 1, **characterised in that** the membrane proteins are proteins which have two or more transmembrane passages.

3. Use according to Claim 1 or 2, **characterised in that** the biological samples are tissue, cells, cell cultures and/or body fluids, bacteria, fungi, viruses or plants.

4. Method for the extraction of membrane proteins from biological samples, **characterised in that** a mixture comprising at least water and between 0.02 and 5% by weight of an ionic liquid is added to a biological sample, optionally with mechanical action, **characterised in that** the ionic liquid is selected from N-(3-hydroxypropyl)-N-methylmorpholinium bistrifluoromethylsulfonylimide, 4-(2-methoxyethyl)-4-methylmorpholinium bromide, 4-methyl-4-propylmorpholinium bromide, 4-(2-ethoxyethyl)-4-methylmorpholinium bromide, 4-(3-hydroxypropyl)-4-methylmorpholinium bromide, 4-(2-hydroxyethyl)-4-methylmorpholinium bromide, 4-(3-methoxypropyl)-4-methylmorpholinium bromide, 4-butyl-4-propylmorpholinium bromide, trihexyl(tetradecyl)-phosphonium tetrafluoroborate, 1-decyl-3-methylimidazolium bromide, 1-dodecyl-3-methylimidazolium chloride, 3-methyl-1-octadecylimidazolium hexafluorophosphate or mixtures thereof.

5. Method according to Claim 4, **characterised in that** the biological sample is lysed in advance.

6. Method according to Claim 4 or 5, **characterised in that** the lysing of the biological samples is carried out by addition of detergents, surface-active substances or pore formers.

7. Method according to one or more of Claims 4 to 6, **characterised in that** the mechanical action is effected by shaking or stirring.

8. Method according to one or more of Claims 4 to 7, **characterised in that** the extraction is carried out at temperatures of 4 to 37°C.

## Revendications

1. Utilisation de mélanges comprenant au moins de l'eau et entre 0,02 et 5% en poids d'un liquide ionique pour l'extraction de protéines membranaires à partir d'échantillons biologiques, **caractérisée en ce que** le liquide ionique est choisi parmi le N-(3-hydroxypropyl)-N-méthylmorpholinium bistrifluorométhylsulfonylimide, le bromure de 4-(2-méthoxyéthyl)-4-méthylmorpholinium, le bromure de 4-méthyl-4-propylmorpholinium, le bromure de 4-(2-éthoxyéthyl)-4-méthylmorpholinium, le bromure de 4-(3-hydroxypropyl)-4-méthylmorpholinium, le bromure de 4-(2-hydroxy-éthyl)-4-méthylmorpholinium, le bromure de 4-(3-méthoxypropyl)-4-méthylmorpholinium, le bromure de 4-butyl-4-propylmorpholinium, le tétrafluoroborate de trihexyl(tétradécyl)phosphonium, le bromure de 1-décyl-3-méthylimidazolium, le chlorure de 1-dodécyl-3-méthylimidazolium, l'hexafluorophosphate de 3-méthyl-1-octadécylimidazolium ou des mélanges de ceux-ci.

2. Utilisation selon la revendication 1, **caractérisée en ce que** les protéines membranaires sont des protéines ayant deux, ou plus, passages trans-membranaires.

3. Utilisation selon la revendication 1 ou 2, **caractérisée en ce que** les échantillons biologiques sont des tissus, des cellules, des cultures cellulaires et/ou des fluides corporels, des bactéries, des champignons, des virus ou des plantes.

4. Méthode d'extraction de protéines membranaires à partir d'échantillons biologiques, **caractérisée en ce qu'**un mélange comprenant au moins de l'eau et entre 0,02 et 5% en poids d'un liquide ionique est ajouté à un échantillon biologique, éventuellement avec une action mécanique, **caractérisée en ce que** le liquide ionique est choisi parmi le N-(3-hydroxypropyl)-N-méthylmorpholinium bistrifluorométhylsulfonylimide, le bromure de 4-(2-méthoxyéthyl)-4-méthylmorpholinium, le bromure de 4-méthyl-4-propylmorpholinium, le bromure de 4-(2-éthoxyéthyl)-4-méthylmorpholinium, le bromure de 4-(3-hydroxypropyl)-4-méthylmorpholinium, le bromure de 4-(2-hydroxyéthyl)-4-méthylmorpholinium, le bromure de 4-(3-méthoxypropyl)-4-méthylmorpholinium, le bromure de 4-butyl-4-propylmorpholinium, le tétrafluoroborate de trihexyl(tétra-décyl)-phosphonium, le bromure de 1-décyl-3-méthylimidazolium, le chlorure de 1-dodécyl-3-méthylimidazolium, l'hexafluorophosphate de 3-méthyl-1-octadécylimidazolium ou des mélanges de ceux-ci.

5. Méthode selon la revendication 4, **caractérisée en ce que** l'échantillon biologiques est lysé à l'avance.

6. Méthode selon la revendication 4 ou 5, **caractérisée en ce que** la lyse des échantillons biologiques est effectuée par l'addition de détergents, de substances tensioactives ou de formateurs de pores.

7. Méthode selon l'une ou plusieurs parmi les revendications 4 à 6, **caractérisée en ce que** l'action mécanique est effectuée par secouage ou agitation.

8. Méthode selon l'une ou plusieurs parmi les revendications 4 à 7, **caractérisée en ce que** l'extraction est effectuée à des températures allant de 4 à 37°C.
